# EUROPEAN PATENT APPLICATION

(11) **EP 2 698 126 A1**
(43) Date of publication of application: **19.02.2014**
(21) Application number: 13179713.6
(22) Date of filing: 08.08.2013
(51) Int. Cl.: A61F 2/06, A61F 2/915, A61M 1/36, A61M 39/04, A61F 2/07, A61M 39/02

(54) **Stent and artificial vessel having the same**

(30) Priority: 14.08.2012 KR 20120088908
(71) Applicant: Lee, Jong-Hoon, Seoul 137-764 (KR)
(72) Inventor: Lee, Jong-Hoon, Seoul 137-764 (KR)
(74) Representative: Grünecker, Kinkeldey, Stockmair & Schwanhäusser

(57) **Abstract**

Disclosed herein is a stent (110) which is inserted into a blood vessel of a patient who has to periodically undergo hemodialysis. The stent includes a wire frame (130) which has a hollow cylindrical structure, and a window (14) which is formed in a predetermined portion of a circumferential surface of the wire frame. The window has no wire therein. In an embodiment, the stent may further a graft which covers the wire frame and the window. The present invention can solve problems not only of deformation of the stent graft which occurs when a hemodialysis needle is inserted into the stent in the conventional technique, but also of patient discomfort when having skin punctured high cost, thrombopoiesis, a risk of recurrence, etc.

## Description

### BACKGROUND OF THE INVENTION

### 1. Field of the Invention

The present invention relates to a stent provided with a window, and an artificial vessel having the stent.

### 2. Description of the Related Art

Maintenance of function of the arteriovenous fistula is so indispensable to renal failure patients who are undergoing maintenance hemodialysis that it is sometimes called a lifeline. However, there is no arteriovenous fistula which over the course of a patient's life can be used problem-free once the operation is performed. Typically, an arteriovenous fistula leads to complications in the long-term. A false or true aneurismal change causes a lot of problems in terms of not only appearance but also blood flow. Moreover, an aneurismal change may cause massive hemorrhage resulting from rupture, so that it may threaten a patient's life. Therefore, such an aneurismal change has to be treated. As for treatment methods, surgical treatment and minimally invasive treatment, for example, inserting a stent graft into a blood vessel of a patient, are representative examples.

However, even if such a stent graft is inserted into the body of a renal failure patient, the patient must regularly undergo hemodialysis.

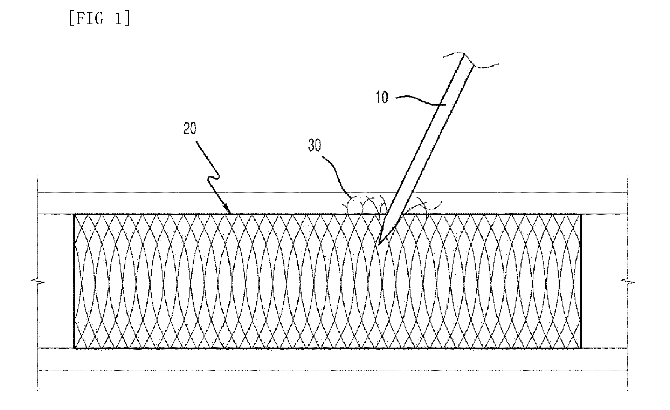

To conduct hemodialysis, a hemodialysis needle 10 which is comparatively thick should be punctured on a stent graft 20. Here, as shown in FIG. 1, a problem that frequently occurs is that wire frame 30 of the stent graft 20 is deformed when frequently inserting a hemodialysis needle 10 into the stent graft 20.

### SUMMARY OF THE INVENTION

Accordingly, the present invention has been made keeping in mind the above problems occurring in the prior art, and an object of the present invention is to provide a stent and an artificial vessel having the stent which is configured such that even if repeated puncture by means of a hemodialysis needle occurs, a wire frame of the stent can be prevented from being deformed.

In order to accomplish the above object, based on TRIZ that is a problem-solving theory derived from the study of patterns of invention, the inventor of the present invention defined the above-stated object as follows.

"To prevent a wire frame from being deformed when conducting a needle puncture for hemodialysis, there must be no wire frame in physical terms, but the wire frame is essential to ensure the function of the stent."

In other words, the wire frame must be present despite the problems associated with it. According to TRIZ, this is physical contradiction. The inventor of the present invention gives attention to "separation in space" of principles of separation which are principles of solution of the physical contradiction, and to "local quality" of forty kinds of principles (40 principles). Therefore, the inventor has devised a stent and an artificial vessel having the stent, which is defined by the accompanying claims, using notions "separation in space" and "local quality."

In an aspect, the present invention provides a stent, including: a wire frame having a hollow cylindrical structure; and a window formed in a predetermined portion of a circumferential surface of the wire frame, the window having no wire therein. The stent may further include an outer covering which covers the wire frame and the window.

The predetermined portion of the circumferential surface of the wire frame may be a portion of an upper half area or a lower half area of the wire frame. A portion of the outer covering that covers the window may be thicker than a portion of the outer covering that covers the wire frame other than the window. The outer covering may comprise a graft.

In another aspect, the present invention provides an artificial vessel, including: a stent comprising a wire frame having a hollow cylindrical structure, and a window formed in a predetermined portion of a circumferential surface of the wire frame, the window having no wire therein; and a graft covering the stent. The predetermined portion of the circumferential surface of the wire frame may be a portion of an upper half area or a lower half area of the wire frame. A portion of the graft that covers the window may be thicker than a portion of the graft that covers the wire frame other than the window. The artificial vessel may further include a marker indicating a location of the window. The marker may be made of a radio-opaque material. In a further aspect, the present invention provides a puncture method, including: disposing the stent according to any one of claims 1 through 4 in a blood vessel of a patient using a dispenser; and puncturing a skin of the patient using a needle and inserting the needle into the stent in such a way that the needle passes through the window of the stent. In still another aspect, the present invention provides a puncture method, including: disposing the artificial vessel according to any one of claims 6 through 8 in a blood vessel of a patient using a dispenser; and puncturing a skin of the patient using a needle and inserting the needle into the stent in such a way that the needle passes through the window of the stent.

According to the present invention, the function of arteriovenous fistula of a patient who has to periodically undergo hemodialysis can be improved, and a patency rate is increased, whereby hemodialysis can be smoothly conducted.

As such, the present invention can solve problems not only of deformation of the stent graft which occurs when a hemodialysis needle is inserted into the stent in the conventional technique, but also of patient discomfort when having skin punctured, high cost, thrombopoiesis, a risk of recurrence, etc.

The effects of the present invention are not limited to the above-stated effects, and those skilled in the art will clearly understand other not mentioned effects from the accompanying claims.

### BRIEF DESCRIPTION OF THE DRAWINGS

The above and other objects, features and advantages of the present invention will be more clearly understood from the following detailed description taken in conjunction with the accompanying drawings, in which:
FIG. 1 is a view showing a stent graft according to a conventional technique;
FIG. 2 is a perspective view illustrating a stent, according to a first embodiment of the present invention;
FIG. 3 is a plan view illustrating the stent of FIG. 2;
FIG. 4 is a side view illustrating the stent of FIG. 2;
FIG. 5 is a perspective view illustrating an artificial vessel in which a stent is covered with a graft, according to a second embodiment of the present invention;
FIG. 6 is a plan view illustrating the artificial vessel of FIG. 5;
FIG. 7 is a side view illustrating the artificial vessel of FIG. 5;
FIG. 8 is a sectional view taken along line A-A' of FIG. 7;
FIGS. 9A and 9B are perspective views showing an artificial vessel provided with a marker; and
FIG. 10 is a view of an artificial vessel to illustrate a puncture operation.

### DESCRIPTION OF THE PREFERRED EMBODIMENTS

With regard to embodiments of the present invention that are disclosed in this specification, special structural or functional explanation is used only for illustrative purposes, and the embodiments of the present invention can be modified in a variety of forms. Therefore, the present invention must not be interpreted as being limited to the embodiments of this specification.

Given the fact that various modifications of the present invention are possible, preferred embodiments of the present invention will be illustrated in the drawings and explained in this specification. However, these embodiments are not intended to limit the present invention to special forms. Rather, all changes that fall within the bounds of the present invention, or the equivalence of the bounds should be understood to be embraced by the present invention.

Hereinafter, preferred embodiments of the present invention will be described in detail with reference to the attached drawings.

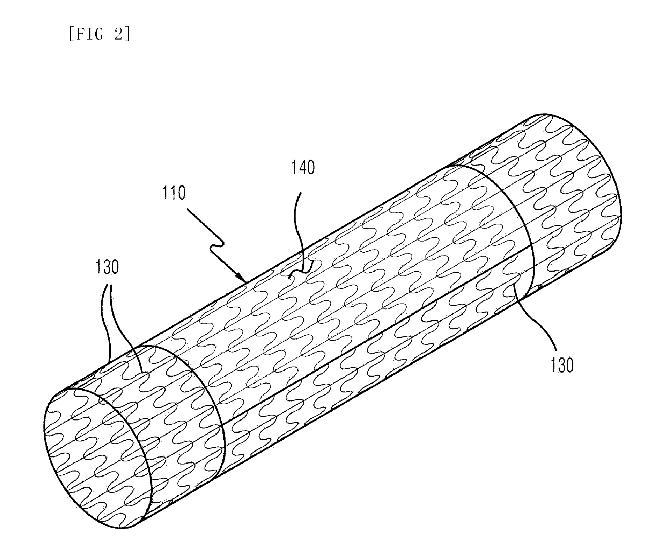

FIG. 2 is a perspective view illustrating a stent according to the present invention. As shown in the drawing, a stent 110 according to a first embodiment of the present invention includes a wire frame 130 and a window 140 which has no wire. The stent 110 has a general cylindrical structure of a predetermined width and length. The wire frame 130 forms a hollow cylindrical structure. The window 140 which has no wire is formed in a predetermined portion of a circumferential outer surface of the wire frame 130. The portion in which window 140 is formed is disposed in an upper half area or a lower half area of the wire frame 130.

In detail, the wire frame 130 includes a plurality of rows of circumferential wires, each of which extends in a circumferential direction in a zigzag shape. The circumferential wires are spaced apart from each other at regular intervals. In portions of the wire frame 130 other than the window 140, each circumferential wire has a circular shape. In a portion of the wire frame 130 in which the window 140 is present, each circumferential wire has a hemispheric shape. Furthermore, the cylindrical wire frame 130 includes a plurality of longitudinal wires, each of which extends a predetermined length in a longitudinal direction, and which are spaced apart from each other at regular intervals. As such, the wire frame 130 forms a hollow cylindrical structure in such a way that the zigzag circumferential wires and the longitudinal wires are connected to each other.

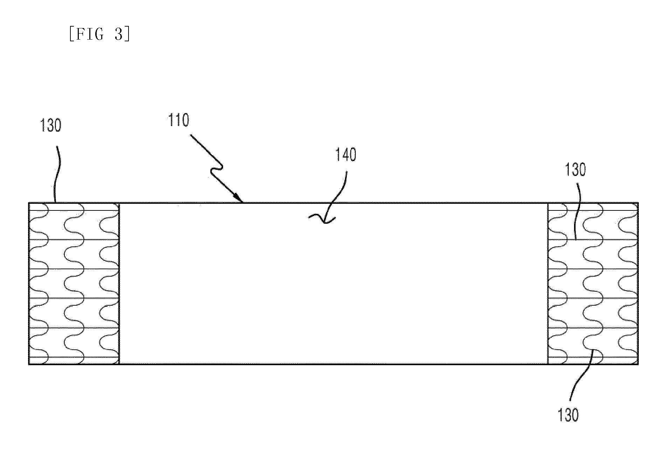

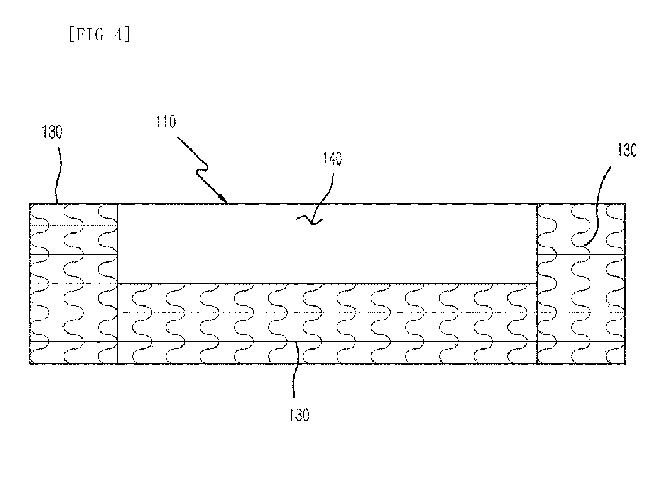

Referring to FIG. 3 showing a plan view of the stent 110, zigzag circumferential wires are provided in opposite ends of the wire frame 130, and the window 140 which has no wire is formed in a medial portion of the wire frame 130. For the sake of understanding, a portion of the wire frame 130 that is disposed just below the window 140 is not shown in FIG. 3. Referring to FIG. 4 showing a side view of the stent 110, the zigzag circumferential wires are provided in the opposite ends of the wire frame 130, and the window 140 is formed in an upper portion of the medial portion of the wire frame 130. As such, the window 140 may be formed in a portion of the upper half area of the wire frame 130, alternatively, it may be formed in a portion of the lower half area of the wire frame 130.

Meanwhile, although the wire frame 130 has predetermined elasticity, it can neither easily be contracted nor expanded by external force. The wire frame 130 maintains a cylindrical shape in the expanded state.

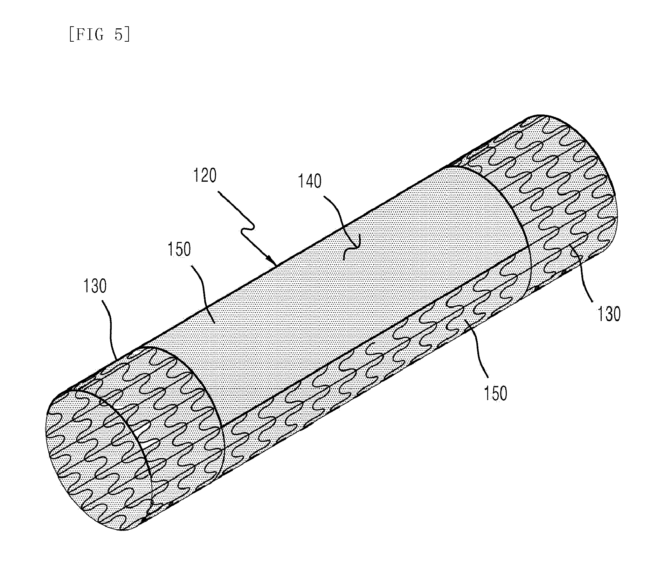

FIG. 5 is a view illustrating an artificial vessel, according to a second embodiment of the present invention. The artificial vessel 120 according to this embodiment comprises a stent 110 of the first embodiment that is covered with an outer covering. The artificial vessel 120 of the second embodiment includes a wire frame 130 and a window 140 which has no wire, that is, substantially has the same elements as those of the stent 110 of the first embodiment. Therefore, in the explanation of the second embodiment, the same reference numerals are used to designate the same or similar elements as those of the first embodiment, and repeated explanation on the construction and function will be omitted.

Referring to FIG. 5, the stent 110 which has a hollow cylindrical structure is covered with an outer covering 150. Preferably, a portion of the outer covering 150 that covers the window 140 is thicker than a portion of the outer covering 150 that covers the wire frame 130 other than the window 140. For reference, the outer covering 150 may be a polytetrafluoroethylene graft. In the same manner as the first embodiment, the stent 110 maintains a circular shape using the structure of the wire frame 130.

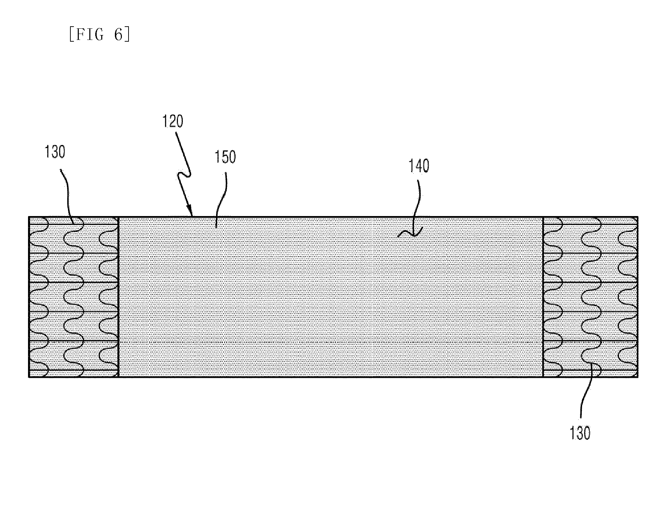

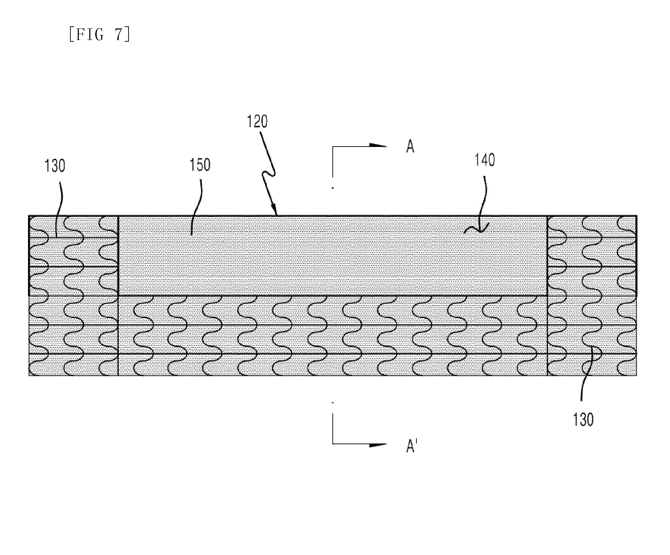

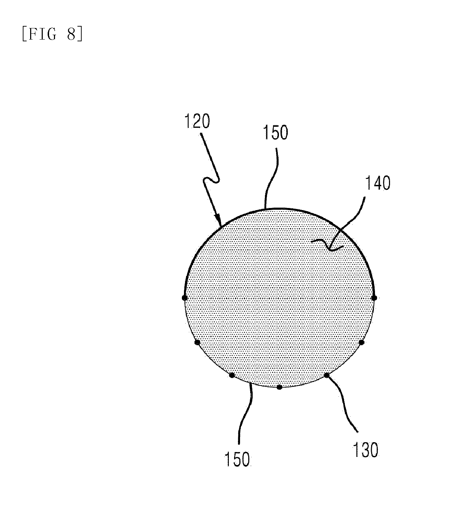

FIG. 6 is a plan view of the artificial vessel of the present invention, and FIG. 7 is a side view of the artificial vessel of the present invention. As shown in the drawings, a plurality of zigzag rows circumferential wires are provided in opposite ends of the wire frame 130, and the window 140 which has no wire is formed in a medial portion of the wire frame 130. The portion of the outer covering 150 that covers the window 140 is thicker than, for example, is twice as thick as the portion of the outer cover 150 that covers the wire frame 130 (refer to FIG. 8). The portion of the outer covering 150 that covers the window 140 is configured to have structural stability such that the artificial vessel 120 which is inserted into a blood vessel 102 of a patient can maintain the same supporting force as that of the wire frame 130 and have durability such that, even if puncture by means of a hemodialysis needle 104 is repeatedly conducted, its shape can be maintained.

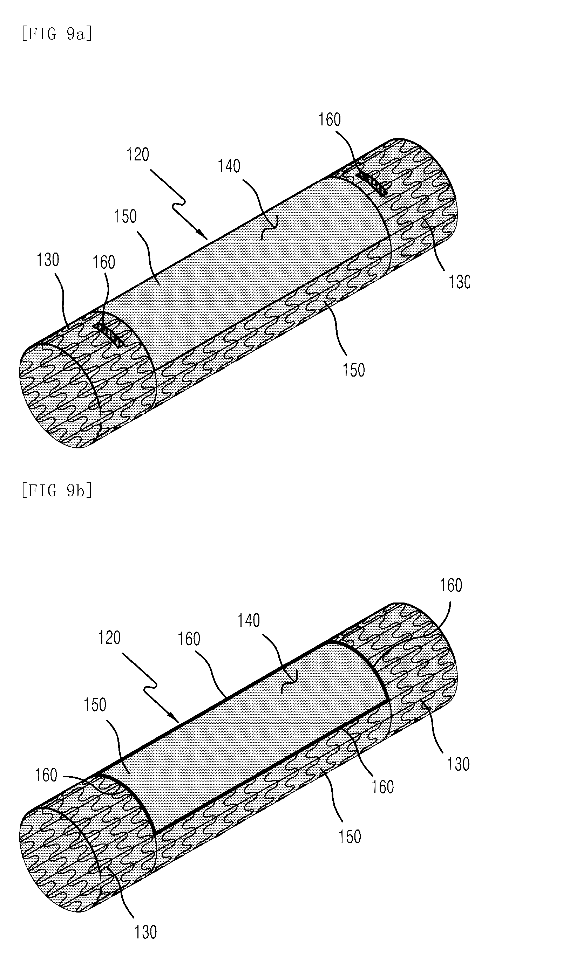

Referring to FIG. 9, to form the stent 110 covered with the outer covering 150, markers 160 may be used to indicate the location of the window 140. The markers 160 are disposed on predetermined portions of the outer covering 150 that corresponds to the window 140. The markers 160 may be preferably printed on the outer covering 150 in a predetermined pattern so that a portion of the outer covering 150 that corresponds to the window 140 can be indicated on the outer surface of the outer covering 150. In another embodiment, the markers 160 may be removably provided on the outer covering 150. Preferably, the markers 160 are disposed adjacent to the window 140 and are made of radio-opaque material so that the markers 160 can be observed on a radiograph. When the markers 160 which are made of radio-opaque material are radiographed, the markers 160 are expressed on a radiograph so that the location of the window 140 can be easily indicated. Given the fact that, when the artificial vessel 120 is inserted into a blood vessel 102, the location of the window 140 may be changed, it is preferable that the location of the window 140 be indicated by the markers 160.

In other words, after the artificial vessel 120 has been disposed in the blood vessel 102, the location of the window 140 in the blood vessel 102 can be easily indicated by the markers 160 using radiography such as fluoroscopy or X-ray graphy. After the location of the window 140 has been discerned, puncture by means of the hemodialysis needle 104 can be conducted through the window 140.

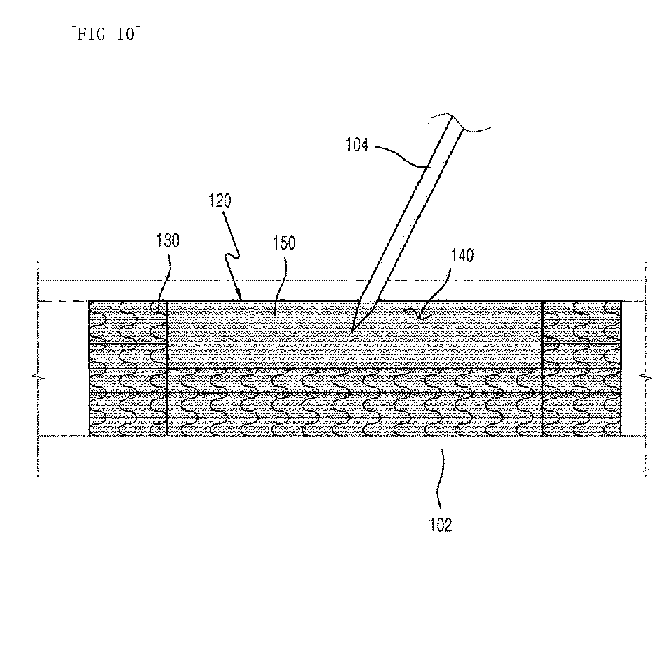

A dispenser is used to insert the stent 110 of the first embodiment or the artificial vessel 120 of the second embodiment into a blood vessel of a patient, particularly, into an arteriovenous fistula of a renal failure patient. After an operation of installing the stent 110 or the artificial vessel 120 has been completed, blood dialysis for the renal failure patient is conducted as follows. Referring to FIG. 10, unlike the conventional technique, the hemodialysis needle 104 can be easily punctured into the stent 110 or artificial vessel 120 only through the window 140 which has no wire. Therefore, even if puncture by means of the hemodialysis needle 104 is repeated several times, the shape of the wire frame 130 of the stent 110 can be prevented from deteriorating.

The fact that the window 140 is formed in the wire frame 130 and the thickness of the outer covering (graft) which covers the wire frame 130 is different between a portion corresponding to the window 140 and the other portion can be interpreted to be technical characteristics of the present invention which use notions of "separation of space" and "partial quality improvement

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

As described above, the present invention can be used to treat an aneurismal change caused on arteriovenous fistula of a patient who must periodically have hemodialysis. The present invention makes it possible for hemodialysis to be reliably conducted.

## Claims

1. A stent, comprising:
a wire frame having a hollow cylindrical structure; and
a window formed in a predetermined portion of a circumferential surface of the wire frame, the window having no wire therein.

2. The stent as set forth in claim 1, further comprising an outer covering covering the wire frame and the window.

3. The stent as set forth in claim 1, wherein the predetermined portion of the circumferential surface of the wire frame is a portion of an upper half area or a lower half area of the wire frame.

4. The stent as set forth in claim 2, wherein a portion of the outer covering that covers the window is thicker than a portion of the outer covering that covers the wire frame other than the window.

5. The stent as set forth in any one of claims 2 through 4, wherein the outer covering comprises a graft.

6. An artificial vessel, comprising:
a stent comprising a wire frame having a hollow cylindrical structure, and a window formed in a predetermined portion of a circumferential surface of the wire frame, the window having no wire therein; and
a graft covering the stent.

7. The artificial vessel as set forth in any one of claims 2 through 6, wherein the predetermined portion of the circumferential surface of the wire frame is a portion of an upper half area or a lower half area of the wire frame.

8. The artificial vessel as set forth in any one of claims 2 through 6, wherein a portion of the graft that covers the window is thicker than a portion of the graft that covers the wire frame other than the window.

9. The artificial vessel as set forth in any one of claims 2 through 6, further comprising
a marker indicating a location of the window.

10. The artificial vessel as set forth in any one of claims 2 through 9, wherein the marker is made of a radio-opaque material.

11. A puncture method, comprising:
disposing the stent according to any one of claims 1 through 4 in a blood vessel of a patient using a dispenser; and
puncturing a skin of the patient using a needle and inserting the needle into the stent in such a way that the needle passes through the window of the stent.

12. A puncture method, comprising:
disposing the artificial vessel according to any one of claims 6 through 8 in a blood vessel of a patient using a dispenser; and
puncturing a skin of the patient using a needle and inserting the needle into the stent in such a way that the needle passes through the window of the stent.
